# EUROPEAN PATENT APPLICATION

(11) **EP 3 564 670 A1**
(43) Date of publication of application: **06.11.2019**
(21) Application number: 18170593.0
(22) Date of filing: 03.05.2018
(51) Int. Cl.: G01N 33/50, G01N 33/574, G01N 33/68

(54) **ANTIBODIES AGAINST TRIM9 AND/OR TRIM67 IN PARANEOPLASTIC NEUROLOGICAL SYNDROMES**

(71) Applicant: Hospices Civils de Lyon, 69002 Lyon (FR); Université Claude Bernard Lyon 1, 69100 Villeurbanne (FR); CENTRE NATIONAL DE LA RECHERCHE SCIENTIFIQUE (CNRS), 75016 Paris (FR); INSTITUT NATIONAL DE LA SANTE ET DE LA RECHERCHE MEDICALE (INSERM), 75013 Paris (FR)
(72) Inventor: DO, Le Duy, 69008 LYON (FR); HONNORAT, Jérôme, 69500 BRON (FR); ROGEMOND, Véronique, 69003 LYON (FR)
(74) Representative: Regimbeau

(57) **Abstract**

The invention relates to a process *in vitro* for diagnosing a paraneoplastic neurological syndrome (PNS) associated with a tumor in an individual, comprising the detection of at least one antibody chosen among antibodies against TRIM9 and antibodies against TRIM67, in a biological fluid of said individual.

SEQUENCE LISTING

<110>  HOSPICES CIVILS LYON
       CNRS
       INSERM
       UNIVERSITE CLAUDE BERNARD LYON 1

<120>  Antibodies against TRIM9 and/or TRIM67 in paraneoplastic
       neurological syndromes

<130>  B74317EP D38159 LUV

<160>  3

<170>  PatentIn version 3.5

<210>  1
<211>  51
<212>  DNA
<213>  Artificial Sequence

<220>
<223>  Oligonucleotide

<400>  1
cggcggtgta gcctggtggc tcccgccagg ctacaccgcc ggggctgcag c                51


<210>  2
<211>  110
<212>  DNA
<213>  Artificial Sequence

<220>
<223>  Oligonucleotide

<400>  2
attcaatggc catctgccag ctgtgcagat ggccattgaa ttcaagtcct cttcagaaag       60

gtgacctagc ctggtggctc ccgccaggct aggtcacctt ggtgagcagc                 110


<210>  3
<211>  58
<212>  DNA
<213>  Artificial Sequence

<220>
<223>  Oligonucleotide

<400>  3
attcaatgaa agagagggag cacaagctct ctttcattga attcaagtcc tcttcaga         58

## Description

### FIELD OF THE INVENTION

The present invention relates to processes of diagnosis of human diseases.

In particular, the present invention relates to a process for diagnosing, in an individual, a paraneoplastic neurological syndrome. The present invention also relates to a process for identifying, in an individual affected by neurological syndrome(s), the presence of a tumor, in particular of a malignant tumor.

### BACKGROUND OF THE INVENTION

Paraneoplastic neurological disorder (PND) or syndrome (PNS) is a rare autoimmune syndrome wherein tumor immunity can recognize central nervous system antigens, triggering degeneration of nervous cells. Antigens common to both the tumor and the nervous system are designated as onconeural antigens.

PNS can affect any part of the central and peripheral nervous system, the neuromuscular junction, and muscle. PNS symptoms can occur isolated or in association. These symptoms are usually severely disabling.

This syndrome is relatively rare, affecting less than 1/10,000 patients with cancer. About 400 cases are diagnosed in France per year.

Mostly, the neurological symptoms precede cancer detection. Therefore, early recognition of PNS can facilitate tumor detection and treatment, and thereby increases the chance of stabilizing the neurological symptoms.

The immune reaction in PNS is often hallmarked by the presence of autoantibodies, directed against onconeuronal antigens, in biological fluids of the patient. The detection of these autoantibodies has proven to be a useful tool in PNS diagnosis.

The first described and the most frequent are anti-Hu and anti-Yo autoantibodies, also named Type 1 antineuronal nuclear antibody (ANNA-1) and anti-Purkinje cell cytoplasmic antibody-type 1 (PCA1). Today, more than 30 other autoantibodies have been described as being associated with PNS (see for review Graus et al., 2014).

Although the role of these antibodies in the pathophysiology of PNS is unclear, they are precious biomarkers allowing prompt diagnosis of a neurological disorder as being a paraneoplastic one. In addition, as these antibodies are usually associated with cancers, they can guide the search for an underlying tumor at a stage when it is frequently not clinically overt.

The patent US 6,387,639 describes the detection of anti-Ma antibodies, in a process for diagnosing a neurologic disorder associated with a neoplasm, such as paraneoplastic limbic encephalitis or brainstem encephalitis.

Recently, autoantibodies against TRIM46, another member of the TRIM family, have been described in three patients having paraneoplastic neurological syndromes, associated with an underlying small-cell lung carcinoma (SCLS) (van Coevorden-Hameete et al., 2017).

Tripartite motif-containing protein is a highly conserved super-family of protein with about 80 members. Antibodies targeting other TRIM proteins were described in other autoimmune diseases, such as antibodies anti-TRIM21 (Ricchiuti et al., 1994) and anti-TRIM38 (Wolska et al., 2016).

Although numerous autoantibodies have already been described in PNS, about one-third of patients have clinical presentation of PNS but do not have detectable known antibodies, suggesting that other antibodies are involved and need to be characterized.

The present invention concerns the identification of novel biomarkers for (i) diagnosing a paraneoplastic neurological syndrome (PNS) in an individual, and (ii) identifying the presence of a tumor in an individual presenting neurological syndromes.

### SUMMARY OF THE INVENTION

Here are described new autoantibodies targeting brain antigens called TRIM9 and TRIM67 in patients with paraneoplastic neurological syndrome, in particular associated with a pulmonary tumor.

The present invention concerns an *in vitro* process for diagnosing a paraneoplastic neurological syndrome (PNS) associated with a tumor in an individual, comprising the detection of at least one antibody chosen among antibodies against TRIM9 and antibodies against TRIM67, in a biological fluid of said individual.

The present invention also concerns an *in vitro* process for identifying the presence of a tumor in an individual affected by at least one neurological syndrome, comprising the detection of at least one antibody chosen among antibodies against TRIM9 and antibodies against TRIM67, in a biological fluid of said individual.

In a specific embodiment of the invention, the tumor is a malignant pulmonary tumor, in particular an adenocarcinoma or a small cell lung cancer (SCLC).

The present invention also concerns an *in vitro* process for monitoring the evolution of a PNS in an individual affected by this syndrome, comprising:
a. determining the amount of at least one antibody chosen among antibodies against TRIM9 and antibodies against TRIM67, in a biological fluid of said individual, at a moment T₀,
b. determining the amount of the same at least one antibody as quantified in step (a), in a biological fluid of said individual, at a moment T₁ later than T₀,
c. comparing the two amounts obtained at T₀ and T₁,
wherein a decrease, between T₀ and T₁, of the amount of said at least one antibody, is indicative of an improvement in the PNS affecting the individual.

The present invention is also related to a kit of diagnostic comprising:
a. at least one antigen or antigen-expressing cell or nucleic acid chosen among the group consisting of:
   i. cells expressing TRIM9 and/or TRIM67 proteins;
   ii. TRIM9 and/or TRIM67 proteins;
   iii. TRIM9 and/or TRIM67 antigenic fragments; and
   iv. an expression vector carrying at least one nucleic acid encoding TRIM9 and/or TRIM67 proteins, or TRIM9 and/or TRIM67 antigenic fragments;
b. anti-human IgG antibody coupled to a probe; and
c. reactants useful for performing the *in vitro* processes as described above.

### DETAILED DESCRIPTION OF EMBODIMENTS OF THE INVENTION

### Definitions

Unless stated otherwise, the following terms and phrases as used herein are intended to have the following meanings:
A syndrome, also designed as a disorder, designates a set of signs and symptoms featuring a specific pathology.

Cancer is a disease which is reflected by uncontrolled growth of endogenous cells. The term "cancer" is used to denote the formation of malignant tumors and neoplasms (tumors or carcinomas). Generally, the growth of cancers comprises two stages, namely local tumor growth then the spread of the cancer. Local tumor growth consists of the appearance of a tumor clone from a "transformed" cell, under the action of initiating and promoting carcinogenic agents. Cell proliferation leads to the formation of the tumor. The spread of the cancer from the initial tumor may occur by regional spread and/or by development of secondary tumors referred to as metastases.

A paraneoplastic syndrome (PNS) is a syndrome that is caused by the presence of a tumor in the body, inducing an immune response against said tumoral cells; the paraneoplastic syndrome is therefore not due to the presence of the tumor, but to the presence of antibodies targeting the tumoral cells. These autoantibodies are directed against tumoral antigens but also cross-react with neurons, and therefore might destroy cells of the central nervous system.

It is understood that in the present application, the phrase "PNS associated with a tumor" is used only for clarity and for referencing of the term "tumor" in the claims, since basically, a PNS is always associated with a tumor.

In the sense of the present invention, when the term "tumor" is used, it designates both benign and malignant tumors. The term "underlying tumor" designates a tumor that causes the neurological syndrome(s) affecting the individual having this tumor. The terms "cancer" and "malignant tumor" are used interchangeably.

Clinical presentations of PNS are heterogeneous since they correlate with the autoantigens / autoantibodies involved. Paraneoplastic syndromes are most commonly associated with cancers of the lung, breast, ovaries, or lymphoma.

Patients presenting a PNS associated with an underlying malignant tumor usually have a slower evolution of the cancer, as compared to patients having a malignant tumor but not affected with neurological symptoms.

### Tripartite Motif-containing proteins

TRIM9 and TRIM67 belong to the C-I family of proteins.

TRIM9 was identified as a cytosolic protein widely expressed in brain (Reymond et al., 2001). TRIM9 is widely expressed in embryonic and adult mouse brain. In adult mouse brain, TRIM9 signal was found in the pyramidal cells of hippocampus, the Purkinje cells of the cerebellum and external layer of the cortex. Immunostaining of TRIM9^{-/-} mouse brain confirmed these observations.

TRIM67 shares 65% of amino acid identity with TRIM9. TRIM67 is expressed mainly in the cerebellum of both mouse and human (Yaguchi et al., 2012).

Both protein have E3-ubiquitin ligase activity and play important role in many neuronal processes including axon branching, neuritogenesis and axon guidance. TRIM9 has also been shown as a negative regulator of NF-kB pathway in the brain.

TRIM9 and TRIM67 play crucial role in maintaining neuron homeostasis.

### Process for diagnosing a paraneoplastic neurological syndrome

The present invention concerns a process *in vitro* for diagnosing a paraneoplastic neurological syndrome (PNS) associated with a tumor in an individual, comprising the detection of at least one antibody chosen among antibodies against TRIM9 and antibodies against TRIM67, in a biological fluid of said individual.

In the sense of the invention, an autoantibody designates an antibody targeting self-antigens, i.e. an antibody directed against one or more of the individual's own proteins.

In the present application, the terms "antibody" and "autoantibody" are used interchangeably; indeed, it is understood that in the sense of the invention, anti-TRIM9 and anti-TRIM67 antibodies are autoantibodies, since TRIM9 and TRIM67 are autoantigens expressed both by tumoral cells and by neuronal cells.

In the sense of the invention, the phrase "detection of at least one antibody" means a step of search, by any technique known by the man skilled in the art, in a biological fluid of the individual to be tested, of at least one antibody.

According to the present invention, the at least one searched antibody is chosen among antibodies against TRIM9 and antibodies against TRIM67.

The phrase "at least one antibody" is synonymous of "one or more antibodies". In the present case, three specific cases are considered: (1) antibodies against TRIM9, or (2) antibodies against TRIM67, or (3) both antibodies against TRIM9 and antibodies against TRIM67, are detected in the biological fluid of an individual.

In a preferred embodiment of the invention, the presence of at least one antibody chosen among antibodies against TRIM9 and antibodies against TRIM67, in a biological fluid of an individual, is indicative of the presence of a paraneoplastic neurological syndrome (PNS) in the individual whose biological fluid is tested.

In a specific implementation of the process according to the invention, the *in vitro* process for diagnosing a paraneoplastic neurological syndrome (PNS) associated with a tumor in an individual comprises the following steps:
- assessing the presence of at least one antibody chosen among antibodies against TRIM9 and antibodies against TRIM67, in a biological fluid of said individual; and
- concluding that the individual is affected by a paraneoplastic neurological syndrome (PNS) if at least one antibody chosen among antibodies against TRIM9 and antibodies against TRIM67 is detected in said biological fluid.

In another specific implementation of the process according to the invention, the *in vitro* process for diagnosing a paraneoplastic neurological syndrome (PNS) associated with a tumor in an individual consists in the two steps listed above.

### Process for identifying the presence of a tumor

The present invention relates to an *in vitro* process for identifying the presence of a tumor in an individual affected by a neurological syndrome, comprising the detection of at least one antibody chosen among antibodies against TRIM9 and antibodies against TRIM67, in a biological fluid of said individual.

In the sense of the invention, an individual presenting a neurological syndrome designates an individual presenting at least one neurological symptom such as, for example:
- paralysis,
- muscle weakness,
- instability
- poor coordination,
- loss of sensation,
- seizures,
- confusion,
- pain,
- altered levels of consciousness,
- difficulty with walking and balance,
- dizziness,
- rapid uncontrolled eye movements,
- difficulty swallowing,
- slurred speech,
- memory loss,
- vision problems,
- sleep disturbances,
- dementia, and
- sensory loss in the limbs.

The phrase « at least one neurological symptom » is synonymous of « one or more neurological symptoms ».

These symptoms are assessed and established by neurological examination, by a health provider or clinician.

According to a preferred embodiment of the invention, the presence of at least one of said antibodies is indicative of the presence of a tumor in said individual whose biological fluid is tested.

This diagnosis process is of particular importance for identifying, in a patient affected by a neurological syndrome, an underlying tumor that was previously not diagnosed; indeed, in the cases of PNS, usually the neurological syndrome precedes the identification of the tumor.

After diagnosis of the presence of a tumor, usually at an early stage, anti-cancer treatments can be administered to the individual for treating specifically such cancer.

In a specific implementation of the process according to the invention, the *in vitro* process for identifying the presence of a tumor in an individual affected by a neurological syndrome comprises the following steps:
- assessing the presence of at least one antibody chosen among antibodies against TRIM9 and antibodies against TRIM67, in a biological fluid of said individual; and
- concluding that a tumor is present in said individual if at least one antibody chosen among antibodies against TRIM9 and antibodies against TRIM67 is detected in said biological fluid.

In another specific implementation of the process according to the invention, the *in vitro* process for identifying the presence of a tumor in an individual affected by a neurological syndrome consists in the two steps listed above.

In the implementation of any process according to the invention, it might happen that both antibodies against TRIM9 and TRIM67 are detected in the biological fluid of the individual.

Exploratory epitope mapping (see examples section) showed that these antibodies against TRIM9 and TRIM67 are polyclonal.

### Features of the underlying tumor

PNS affect the central or peripheral nervous system; some are degenerative though others may improve with treatment of the condition or of the tumor. Symptoms of PNS may include difficulty with walking and balance, dizziness, rapid uncontrolled eye movements, difficulty swallowing, loss of muscle tone, loss of fine motor coordination, slurred speech, memory loss, vision problems, sleep disturbances, dementia, seizures, and sensory loss in the limbs.

In some rare cases, PNS might be associated with begnin tumor, such as ovarian teratoma. However, in most cases, the associated tumor is malignant.

In an embodiment of the invention, the diagnosis processes according to the invention are related, respectively, to:
- the diagnosis of PNS associated with a malignant tumor, or
- the identification of a malignant tumor in an individual affected by at least one neurological syndrome; otherwise said, the diagnostic of an individual as being affected by a cancer.

The most common cancers associated with PNS are lymphoma and breast, ovarian, and lung cancers, but many other cancers can produce paraneoplastic symptoms as well.

According to a specific embodiment of the invention, the tumor associated with the PNS is not a melanoma, i.e. a skin malignant tumor.

The publication (Larman *et al*., 2014) is related to a synthetic human proteome, and its use for profiling the antibody repertoires of individuals. In this article, an example is related to a female patient with PNS secondary to melanoma with an unusual clinical presentation (ataxia, dysarthria, horizontal gaze palsy). TRIM9 and TRIM67 autoantigens appear to have been accidentally detected in her cerebrospinal fluid. Nevertheless, no reliable scientific conclusions can be drawn from this labelling whose specificity is not established, since the number of negative controls is too low.

In an embodiment of the invention, the malignant tumors associated with PNS are chosen among the group consisting of: breast tumors, ovarian tumors, lung tumors and lymphoma.

In a specific embodiment of the invention, the malignant tumor is a pulmonary tumor. More particularly, the malignant tumor is a lung adenocarcinoma or a small cell lung cancer.

According to this embodiment, a process for diagnosing a paraneoplastic neurological syndrome (PNS) associated with a pulmonary tumor in an individual comprises the following steps:
- assessing the presence of at least one antibody chosen among antibodies against TRIM9 and antibodies against TRIM67, in a biological fluid of said individual; and
- concluding that the individual is affected by a paraneoplastic neurological syndrome (PNS) associated with a pulmonary tumor if at least one antibody chosen among antibodies against TRIM9 and antibodies against TRIM67 is detected in said biological fluid.

In a specific implementation of the process according to the invention, the *in vitro* process for identifying the presence of a pulmonary tumor in an individual affected by a neurological syndrome comprises the following steps:
- assessing the presence of at least one antibody chosen among antibodies against TRIM9 and antibodies against TRIM67, in a biological fluid of said individual; and
- concluding that a pulmonary tumor is present in said individual if at least one antibody chosen among antibodies against TRIM9 and antibodies against TRIM67 is detected in said biological fluid.

### Features of neurological syndromes

In the sense of the invention, the general term PNS designates and includes multiple neurological syndromes that may be observed to be associated with the presence of a cancer / a tumor in an individual.

The following table 1 lists the main paraneoplastic neurological syndromes, and the identified associated antibodies.

**Table 1. Main paraneoplastic neurological syndromes and associated antibodies**

| **Paraneoplastic neurological syndromes** | **Frequency of paraneoplastic origin** | **Main associated tumors** | **Main frequent associated paraneoplastic antibodies** |
|---|---|---|---|
| LEMS | 60% | SCLC | VGCC-Ab* |
| Subacute cerebellar ataxia | 50% | Ovary, breast | Yo-Ab (PCA1-Ab**) |
| | | SCLC | Hu-Ab (ANNA1-Ab**) |
| | | Hodgkin's disease | CV2-Ab (CRMP5-Ab**) |
| | | Others | Tr-Ab (DNER-Ab**) |
| | | | Ma-Ab (Ta-Ab**) |
| Opsomyoclonus | 20% | Neuroblastoma | Hu-Ab (ANNA1-Ab**) |
| | | Breast, lung | Ri-Ab (ANNA2-Ab**) |
| Sensory neuronopathy | 20% | SCLC | Hu-Ab (ANNA1-Ab**) |
| | | | CV2-Ab (CRMP5-Ab**) |
| Limbic encephalitis | 20% | SCLC | Hu-Ab (ANNA1-Ab**) |
| | | Testicular | CV2-Ab (CRMP5-Ab**) |
| | | SCLC | Ma2-Ab (Ta-Ab**) |
| | | | Amphiphysin-Ab |
| Encephalomyelitis | 10% | SCLC Others | Hu-Ab (ANNA1-Ab**) |
| | | | CV2-Ab (CRMP5-Ab**) |
| | | | Amphiphysin-Ab |
| | | | Ma2-Ab (Ta-Ab**) |
| Retinopathy | NA | SCLC | Recoverin-Ab |
| | | Melanoma | CV2-Ab (CRMP5-Ab**) |
| | | | Rod-bipolar-cell-Ab |
| Stiff-person | 20% | Breast | Amphiphysin-Ab |
| syndrome | | | |
| Chronic gastrointestinal pseudoobstruction | NA | SCLC | Hu-Ab (ANNA1-Ab**) |
| | | | CV2-Ab (CRMP5-Ab**) |

| | | | |
|---|---|---|---|
| **VGCC-Ab are not really paraneoplastic antibodies because they are involved in LEMS but are not independent on the presence of a tumor* ***Alternative nomenclature which can be found in the literature* | | | |

Other neurological syndromes include dementia, brainstem encephalitis, Lambert-Eaton myasthenic syndrome and dermatomyositis/polymyositis.

In particular, the neurological syndrome associated with the tumor is at least one of the following syndromes: subacute cerebellar ataxia, opsoclonus-myoclonus, sensory neuronopathy, limbic encephalitis, encephalomyelitis, stiff-person syndrome, dementia, brainstem encephalitis, Lambert-Eaton myasthenic syndrome and dermatomyositis.

These syndromes are extensively described in the review entitled "Paraneoplastic neurological syndromes", from Honnorat and Antoine, published in 2007 (Orphanet journal of rare diseases).

Subacute cerebellar ataxia is a neurological syndrome characterized by the rapid development of severe pancerebellar dysfunction due to an extensive loss of Purkinje neurons with relative preservation of other cerebellar neurons.

Opsoclonus-myoclonus (OM) is defined by the presence of spontaneous, arrhythmic and large amplitude conjugate saccades occurring in all directions of gaze, without saccadic interval. Opsoclonus is usually associated with myoclonus of the limbs and trunk, and sometimes, with encephalopathy.

Sensory neuronopathy (SSN) is characterized by primary damage of the sensory nerve cell body of the dorsal root ganglia. The main clinical complains at onset are pain and paresthesias with asymmetric distribution that involves the arms rather than the legs. Later, pain is replaced by numbness, limb ataxia, and pseudoathetotic movements of the hands.

Limbic encephalitis (LE) is characterized by the subacute onset of confusion with marked reduction of the short-term memory. Seizures are not uncommon and may antedate by months the onset of cognitive deficit. Other patients have a more insidious onset with depression or hallucinations that can lead to confusion the diagnosis with that of a psychiatric illness. Among LE patients, 50% have small-cell lung cancer (SCLC), 20% have a testicular tumor and 8% have breast cancer.

Encephalomyelitis (EM) is characterized by involvement of different areas such as hippocampus, lower brainstem, spinal cord or dorsal root ganglia in one and the same time. The clinical picture reflects the variable anatomic involvement and includes: LE, brainstem syndromes, autonomic dysfunction, myelitis or SSN. In 75% of patients, the underlying neoplasm is SCLC.

Brainstem encephalitis is a particular encephalitis affecting the brainstem, the cerebellum and the cranial nerves.

Stiff-Person syndrome (SPS) is a neurological disorder characterized by stiffness, more prominent in axial muscles, with co-contraction of agonist and antagonist muscle groups and painful spasms precipitated by sensory stimuli.

Dementia designates a long-term and often gradual decrease in the ability to think and remember, that affects the daily functioning of an individual. Other common symptoms include emotional problems, problems with language, and a decrease in motivation.

Lambert-Eaton myasthenic syndrome (LEMS) is a disorder of the neuromuscular junction characterized by muscle weakness and autonomic dysfunction and, on electromyography, by low compound muscle action potential after nerve stimulation with decrement at low frequency stimulation (3 Hz) of more than 10% and increment after high frequency stimulation (more than 20 Hz or preferably maximal voluntary contraction) of more than 100%. SCLC is the main associated cancer, detected mostly within two years after the diagnosis of LEMS.

Dermatomyositis is an idiopathic inflammatory myopathy with characteristic cutaneous manifestations including heliotrope rash of the periorbital skin, erythematous scaly plaques on dorsal hands with periungual telangiectasia and photosensitive poikilodermatous eruption. The myopathy, designed as polymyositis, is generally symmetrical and slowly progressive during a period of weeks to months affecting mainly the proximal muscles. A paraneoplastic origin is suspected in 30% of patients and the main associated cancer are ovarian, lung, pancreatic, stomach, and colorectal cancers, and non-Hodgkin lymphoma.

Retinopathies include three well-characterized syndromes:
- cancer-associated retinopathy (CAR) is almost always associated with SCLC. Symptoms usually present bilaterally and reflect the simultaneous dysfunction of both cones and rods. Patients present with photosensitivity, reduced visual acuity, decreased color perception, ring scotomas, nyctalopia, and prolonged dark adaptation,
- melanoma-associated retinopathy (MAR) usually occurs after the diagnosis of malignant melanoma, often at the stage of metastases. Patients with MAR usually have nearly normal visual acuity and color vision but develop sudden shimmering, flickering photopsias, night blindness, and mild peripheral visual field loss,
- uveitis, optic neuropathy or retinopathy are also observed in some patients with CV2-Antibodies.

Chronic gastrointestinal pseudoobstruction (CGP) is characterized in patients by a weight loss, persistent constipation and abdominal distension due to damage of the neurons of the enteric plexuses. Some patients may present with dysphagia, nausea and vomiting due to esophageal dysmotility or gastroparesis, or more frequently, with severe constipation. The most commonly associated tumor and antibodies are SCLC and Hu-Ab or CV2-Ab, respectively.

### Antibodies frequently detected in PNS

The table 2 below lists the major autoantibodies that are detected in individuals affected by PNS.

**Table 2. Main antibodies associated with Paraneoplastic neurological syndromes**

| **Antibodies** | **Main associated neurological syndromes** | **Cancer** |
|---|---|---|
| **Hu-Ab (ANNA1-Ab**)** | Sensory neuronopathy | SCLC |
| | Encephalomyelitis | |
| | Chronic gastrointestinal pseudoobstruction | |
| | Cerebellar ataxia | |
| | Limbic encephalitis | |
| **Yo-Ab (PCA1-Ab**)** | Subacute cerebellar ataxia | Ovary, breast, uterus |
| **CV2-Ab (CRMP5-Ab**)** | Cerebellar ataxia | SCLC, thymoma |
| | Sensory-motor neuropathy | |
| | Uveitis, retinopathy | |
| | Encephalomyelitis | |
| **Ri-Ab (ANNA2-Ab**)** | Opsomyoclonus | Breast, SCLC |
| | Cerebellar ataxia | |
| **Amphiphysin-Ab** | Stiff-person syndrome | Breast, SCLC |
| | Sensory neuronopathy | |
| | Encephalomyelitis | |
| **Tr-Ab (DNER-Ab**)** | Cerebellar ataxia | Hodgkin's disease |
| **Ma2-Ab (Ta-Ab**)** | Limbic encephalitis | Testicular |
| **CAR-Ab** | Retinopathy | Breast, SCLC |

| | | |
|---|---|---|
| ***Alternative nomenclature which can be found in the literature* | | |

In a specific embodiment of the invention, the process comprises a preliminary step consisting in a step of detection of at least one antibody chosen among the group consisting of: antibodies anti-Yo, anti-Hu, anti-CV2/CRMP5, anti-Ri and anti-Ma2.

According to this embodiment, an *in vitro* process for diagnosing a paraneoplastic neurological syndrome (PNS) associated with a tumor in an individual comprises the following steps:
a. assessing the presence of at least one antibody chosen among the group consisting of: antibodies anti-Yo, anti-Hu, anti-CV2/CRMP5, anti-Ri and anti-Ma2, in a biological fluid of said individual;
b. assessing the presence of at least one antibody chosen among antibodies against TRIM9 and antibodies against TRIM67, in a biological fluid of said individual; and
c. concluding that the individual is affected by a paraneoplastic neurological syndrome (PNS) if at least one antibody chosen among antibodies against TRIM9 and antibodies against TRIM67 is detected in said biological fluid,
with the steps (a) and (b) being carried out one after another, or being carried out concomitantly.

Similarly, according to this embodiment, an *in vitro* process for identifying the presence of a tumor in an individual affected by a neurological syndrome comprises the following steps:
a. assessing the presence of at least one antibody chosen among the group consisting of: antibodies anti-Yo, anti-Hu, anti-CV2/CRMP5, anti-Ri and anti-Ma2, in a biological fluid of said individual;
b. assessing the presence of at least one antibody chosen among antibodies against TRIM9 and antibodies against TRIM67, in a biological fluid of said individual; and
c. concluding that a tumor is present in said individual if at least one antibody chosen among antibodies against TRIM9 and antibodies against TRIM67 is detected in said biological fluid,
with the steps (a) and (b) being carried out one after another, or being carried out concomitantly.

In another embodiment of the invention, a supplementary step for assessing the presence of antibodies against TRIM46 and/or against TRIM21 is performed, concomitantly or not.

In a preferred embodiment of the invention, the antibodies chosen among the group consisting of: antibodies anti-Yo, anti-Hu, anti-CV2/CRMP5, anti-Ri and anti-Ma2 are undetectable in the biological fluid of the individual.

In another embodiment of the invention, antibodies against TRIM46 and/or against TRIM21 are undetectable in the biological fluid of the individual.

### Biological fluid

The term "biological fluid of said individual" is intended to mean specifically a sample of biological fluid taken from said individual, chosen from the group consisting of: cerebrospinal fluid (CSF), serum, whole blood, urine, lymph, saliva, sputum and tears.

Preferentially, the biological fluid from the individual is cerebrospinal fluid (CSF).

The man skilled in the art knows the appropriate technique for the sampling of a convenient amount of biological fluid from an individual, for the implementation of a process according to the invention.

### Techniques of detection of antibodies in a biological fluid

The processes according to the invention comprise a step of detection of antibodies in a biological fluid.

A plurality of techniques is available to assess the presence of a specific antibody in a fluid. For example, the following techniques might be used:
a. enzyme-linked immunosorbent assay (ELISA), Immuno-electrophoresis, Western blot, dot-blot,
b. immunostaining of antigen-expressing tissues, such as immunohistochemistry; and
c. cell-based assay, based on the use of living cells expressing antigens.

In a specific embodiment of the invention, the detection of antibodies is performed according to at least one of the following techniques: immunoblotting such as western blot or dot-blot, immunohistochemistry, ELISA and cell-based assay.

### Quantification of antibodies

In a specific implementation of the process according to the invention, the detection of at least one antibody comprises furthermore the quantification of said at least one antibody in the biological fluid.

Techniques of detection of antibodies as listed above can be used for quantifying or semi-quantifying the amount of the at least one antibody chosen among antibodies against TRIM9 and antibodies against TRIM67, and also for quantifying or semi-quantifying the amount of the at least one antibody chosen among antibodies anti-Yo, anti-Hu, anti-CV2/CRMP5, anti-Ri, anti-Ma2, anti-TRIM46 and anti-TRIM21.

In particular, a quantification process may comprise the following steps:
a. determining a value corresponding to the amount of at least one antibody chosen among antibodies against TRIM9 and antibodies against TRIM67, in a biological fluid from an individual, by techniques of detection and quantification, and
b. comparing the value obtained in step a) with a value representative of a specific amount, for example:
   i. the amount of antibodies against TRIM9 in an individual known to be affected by a PNS;
   ii. the amount of antibodies against TRIM67 in an individual known to be affected by a PNS;
   iii. the amount of antibodies against TRIM9 and of antibodies against TRIM 67 in an individual known to be affected by a PNS;
   iv. the amounts of antibodies against TRIM9 and/or against TRIM67 measured in at least two individuals known to be affected by a PNS.

Alternatively, the biological fluid is diluted at different rates of dilution, and each of the obtained results or immunostaining pictures are compared to results or pictures representatives of a specific amount of antibody.

For quantifying the antibodies, the preferred technique is the technique of "end point dilution". The "end point dilutione is the dilution ratio of the tested sample where a positive signal, according to the used technique, is not detectable anymore.

A reference value will be determined by those skilled in the art, in function of the measure and process.

Main advantages of quantifying the amount of circulating antibodies against TRIM9 and/or antibodies against TRIM67 are:
- the possibility of following / monitoring the evolution of a PNS,
- the possibility to compare several individuals affected by a PNS, presenting different clinical pictures,
- eventually, to identify a correlation between certain amounts of antibodies and specific clinical pictures.

### Process of monitoring

The present invention also concerns a process for monitoring the evolution of a PNS in an individual affected by this syndrome, comprising the following steps:
a. determining the amount of at least one antibody chosen among antibodies against TRIM9 and antibodies against TRIM67, in a biological fluid of said individual, at a moment T₀;
b. determining the amount of the same at least one antibody as quantified in step (a), in a biological fluid of said individual, at a moment T₁ later than T₀;
c. comparing the two amounts obtained at T₀ and T₁,
wherein a decrease, between T₀ and T₁, of the amount of said at least one antibody, is indicative of an improvement in the PNS affecting the individual.

Such improvement may be for example a decrease of the neurological symptoms, a reduction of the size of the tumor, or a reduction of the aggressiveness of the tumor.

It is understood that the biological fluids from the individuals are removed from the individuals at these moments T₀ and T₁, T₁ being later than T₀, and that the determined amounts correspond to the state of the individual at these moments T₀ and T₁, even if the measures and comparisons are realized extemporarily.

More specifically, the present invention relates to an *in vitro* process for determining the efficacy of a treatment for a PNS in an individual affected by this syndrome, comprising the following steps:
a. determining a value corresponding to the amount of at least one antibody chosen among antibodies against TRIM9 and antibodies against TRIM67, in a biological fluid from said individual before the start of said treatment (T₀),
b. determining the value corresponding to the amount of the same at least one antibody as quantified in step(a) in a biological fluid from said individual after the start of said treatment (T₁), and
c. comparing the two values obtained at T₀ and T₁,

it being understood that said treatment is judged to be effective when the T₀ value determined in step a) is greater than the T₁ value determined in step b).

Such a process which makes it possible to monitor the progression and/or the efficacy of a treatment corresponds to what can be referred to as a "companion test" which makes it possible to adjust or change a treatment, according to the specific response of a given individual to this treatment, during said treatment.

### Kit of diagnostic

The present invention also relates to a kit of diagnostic comprising:
a. at least one antigen or antigen-expressing cell or nucleic acid chosen among the group consisting of:
   i. cells expressing TRIM9 and/or TRIM67 proteins;
   ii. TRIM9 and/or TRIM67 proteins;
   iii. TRIM9 and/or TRIM67 antigenic fragments; and
   iv. an expression vector carrying at least one nucleic acid encoding TRIM9 and/or TRIM67 proteins, or TRIM9 and/or TRIM67 antigenic fragments;
b. anti-human IgG antibody coupled to a probe; and
c. reactants useful for performing the *in vitro* processes such as described above.

This kit is adapted for the implementation of any *in vitro* process such as described above.

Concerning the cells expressing TRIM9 and/or TRIM67 proteins, the man skilled in the art will determine which cell lines are the most adapted host for expressing these proteins. Preferentially, these cells have an human origin. As an example, HEK293 cells, derived from human embryonic kidney, are suitable for being part of this kit.

To express TRIM9 and/or TRIM67 proteins, cells can be transfected with expression vectors (plasmids) carrying at least one nucleic acid encoding TRIM9 and/or TRIM67 proteins or antigenic fragments thereof, optionally tagged to be detected easily.

These TRIM9 or TRIM67 expressing cells are suitable for performing a cell-based assay in the context of the implementation of the processes as described above.

TRIM9 and/or TRIM67 proteins can also be used in this kit, and are in particular suitable for performing western-blot and dot-blot assays.

In the sense of the invention, "TRIM9 antigenic fragments" and "TRIM67 antigenic fragments" designate portions of the whole proteins that contain the epitope, i.e. the part of the protein that is recognized by antibodies.

"TRIM9 antigenic fragments" and "TRIM67 antigenic fragments" are also suitable for performing western-blot and dot-blot assays.

The kit according to the invention also contains an anti-human IgG antibody, also designed as a "secondary antibody" since its goal is the detection of a primary antibody, targeting a specific antigen. This anti-IgG antibody is coupled to a probe such as Alexa Fluor dyes or horseradish peroxidase (HRP), which enable detection of the secondary antibody.

Probes can be for example fluorescent probes, such as Alexa555.

The kit according to the invention also contains useful reactants: for example, the kit might contain reactants adapted for the detection of the probe coupled to the anti-IgG antibody.

### Treatment strategies for paraneoplastic neurological syndrome

Treatment options for PNS include:
- therapies to eliminate the underlying cancer, such as chemotherapy, radiation and surgery; and
- therapies to eliminate the autoantibodies and related lymphocytes, to limit the neuronal degeneration, with non-specific immunomodulatory treatments.

By using non-specific immunomodulatory compounds, the immune reaction can be controlled and the neurological symptoms might decrease or at least stabilize.

Both treatment options may be performed concomitantly or sequentially.

The present invention also relates to a method of diagnosis of a paraneoplastic neurological syndrome (PNS) in an individual susceptible to be affected by such syndrome, comprising the following steps:
- in a biological fluid issued from said individual, assessing the presence of at least one antibody chosen among antibodies against TRIM9 and antibodies against TRIM67,
- if at least one of said antibodies is present in said biological fluid, the individual is diagnosed as being affected by a PNS.

The present invention also relates to a method of treatment of a paraneoplastic neurological syndrome (PNS) comprising the following steps:
- in a biological fluid issued from an individual susceptible to be affected by such syndrome, assessing the presence of at least one antibody chosen among antibodies against TRIM9 and antibodies against TRIM 67,
- if at least one of said antibodies is present in said biological fluid, the individual is diagnosed as being affected by a PNS,
- treating said individual affected by a PNS with:
   ∘ therapies to eliminate the underlying cancer, such as chemotherapy, radiation and surgery; and/or
   ∘ therapies to eliminate the autoantibodies and related lymphocytes.

The present invention also relates to a method of diagnosis of a cancer in an individual affected by a neurological syndrome, comprising the following steps:
- in a biological fluid issued from said individual, assessing the presence of at least one antibody chosen among antibodies against TRIM9 and antibodies against TRIM 67,
- if at least one of said antibodies is present in said biological fluid, the individual is diagnosed as being affected by a cancer.

The present invention also relates to a method of treatment of a cancer comprising the following steps:
- in a biological fluid issued from an individual affected by at least one neurological syndrome, assessing the presence of at least one antibody chosen among antibodies against TRIM9 and antibodies against TRIM 67,
- if at least one of said antibodies is present in said biological fluid, the individual is diagnosed as being affected by a cancer,
- treating said individual affected by a cancer associated with at least one neurological syndrome with:
   ∘ therapies to eliminate the underlying cancer, such as chemotherapy, radiation and surgery; and/or
   ∘ therapies to eliminate the autoantibodies and related lymphocytes.

As previously stated, the most common cancers associated with PNS are lymphoma and breast, ovarian, and lung cancers, but many other cancers can produce paraneoplastic symptoms as well.

According to a specific embodiment of the invention, the cancer associated with the PNS is not a melanoma, i.e. a skin malignant tumor.

In an embodiment of the invention, the cancer associated with PNS is chosen among the group consisting of: breast tumors, ovarian tumors, lung tumors and lymphoma.

In a specific embodiment of the invention, the cancer is a pulmonary cancer. More particularly, the cancer is a lung adenocarcinoma or a small cell lung cancer.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### Example 1. Identification of TRIM9 and TRIM67 as autoantibodies targets in paraneoplastic neurological syndromes

Between October 2007 and June 2016, more than 8000 samples of cerebrospinal fluid (CSF) from patients suspected of autoimmune encephalitis or paraneoplastic neurological syndromes were tested in the French reference center. Among them, 32 samples with autoantibodies directed against uncharacterized neuronal targets, and issued from patients with clinical presentation highly suggestive of PCD (paraneoplastic cerebellar degeneration, PCD group), were identified by immunohistochemistry.

To perform future characterization, samples of these patients were deposited in the collection of biological samples named "NeuroBioTec", Biobank of the Hospices Civils de Lyon (France, AC-208-73, NFS96-900).

Written consent was obtained from all patients with the approval of the Institutional Review Board of the University Claude Bernard Lyon 1 and Hospices Civils de Lyon.

All the clinical and paraclinical data of these patients were collected by study investigators and clinical research associates at the time of PCD diagnosis and during follow up.

Among the 32 samples with autoantibodies directed against uncharacterized neuronal targets, a first patient's CSF was identified as showing a particular reactivity with the cytoplasm of neurons and neuropil of the cerebellum, hippocampus, and cerebral cortex, that was different from known autoantibodies associated with PCD.

This sample of the first patient is hereafter designed as "CSF1".

Indirect immunohistochemistry on rat brain section showed a strong staining of cell bodies and dendrites of CA3 neurons in the hippocampus (Table 3). In the cerebellum, marked immunolabelling was observed in Purkinje's cell bodies and dendrites (Table 3).

**Table 3. Immunohistochemistry performed on rat brain section (12 µm thickness, sagittal section) with CSF1 and control CSF, showing particular staining pattern**

| | Cerebellum | | | Hippocampus | | | Cortex |
|---|---|---|---|---|---|---|---|
| | Granular layer | Molecular layer | Purkinje cell layer | CA1 cells | CA3 cells | Dentate Gyrus | |
| CSF 1 | + | + | + | + | + | + | + |
| Control CSF | - | - | - | - | - | - | - |

Immunostaining obtained with fixed and permeabilized murine neuron shows a strong labeling of cytosolic clusters (Table 4).

**Table 4. Subcellular localization of the antigen**

| Cytosol | Membrane | Nucleus |
|---|---|---|
| + | - | - |

The clinical presentation of **Patient 1 is the following:** A 65 years old man, with past medical history characterized by 80 pack-years of cigarettes and pulmonary right lobar well differentiated adenocarcinoma Stage 1B T2N0, diagnosed 2 years before and treated by surgery only. He developed sub-acutely, in less than 1 month, an isolated and severe cerebellar ataxia. The patient was unable to walk without support and developed a dysarthria. Examination revealed a severe static cerebellar syndrome with extension of the support polygon and inability to stand up without help. A mild dysmetria and hypermetria were also observed predominating on the left side. No motor, sensitive or cognitive deficit was observed. Brain MRI was normal and the CSF showed inflammation (Prot: 0.41 g/l; Lymphocytes 9/mm3 with activated lymphoplasmocytes; few oligoclonal bands (OCB) and an IgG index of 0.65 (normal <0.7)). A fluorodeoxyglucose body Pet-scan showed a mediastinal highly metabolic lymph node of 1.5 cms suggesting a metastatic evolution of the pulmonary adenocarcinoma. The patient refused more investigation and was treated by mediastinal radiotherapy. Twelve years later, the patient is still alive with a stable but severe cerebellar ataxia and without recurrence of his cancer.

Western-blots using rodent brain protein extract show that CSF1 recognizes two specific protein band at ∼72 and 95 kDa (see results in table 6).

No labeling has been observed on mice living neuron (data not shown), suggesting that the autoantibodies targeted exclusively intracellular proteins.

To identify the antigen, whole mouse brain homogenate was incubated with CSF1, and the antigen was immuno-precipitated. Subsequent mass spectrometry analyses show that TRIM9 and TRIM67 are specifically enriched in the 95-kDa band and that the 72-kDa band contains also TRIM9 but not TRIM67.

To confirm the presence of anti-TRIM9 and TRIM67-antibodies in CSF1, HEK293 cells were transfected respectively with full-length cMyc-tagged TRIM9 or TRIM67 plasmids (pCS2-TRIM9-Myc / pCS2-TRIM67-Myc). After 24h of culture, cells were fixed in paraformaldehyde (PFA) 4% during 15 minutes. Cells were then incubated with the patients CSF at a dilution rate of 1/100. A secondary antibody anti-human IgG, coupled to a fluorescent probe Alexa555, is used for detecting presence of autoantibodies against TRIM9 and/or TRIM67 demonstrating that CSF1 recognizes both TRIM9 and TRIM67, although the control CSF does not (see results in table 7).

Specific staining of TRIM9 and TRIM67-expressing cells prove the presence of anti-TRIM9/67 antibodies in CSF1.

Then, another patient having TRIM9/TRIM67 antibodies in her CSF (patient 2) was identified among the 32 patients of PCD group..

The clinical presentation of **Patient 2 is the following:** A 78 years old woman, with past medical history characterized by 30 pack-years of cigarettes and diabetes mellitus type II treated by diet. She developed sub-acutely, in less than 1 month, a severe cerebellar ataxia. The patient was unable to walk without support. Examination revealed dysarthria and a severe static cerebellar syndrome with extension of the support polygon and inability to stand up without help. Dysmetria and hypermetria were severe in the inferior limbs. No motor or sensitive deficit was observed, but she had cognitive impairment with anterograde amnesia and dysexecutive symptoms (Minimental scale was 20/30 and Frontal Assessment Battery 8/18). Brain MRI was normal and the CSF showed inflammation (Prot: 0.65 g/l; Lymphocytes 17/mm3; more than 10 OCB and an IgG index of 1.52 (normal <0.7)). A fluorodeoxyglucose body Pet-scan showed a mediastinal highly metabolic lymph nodes and a cytologic punction demonstrated the presence of a poorly differentiated adenocarcinoma (CK7 and TTF1 positive, without EGFR, KRAS, BRAF and ERBB2 mutation). The patient was treated by chemotherapy (Carboplatine and Taxol), corticosteroids and immunoglobulins without effect on the neurological symptoms and with a partial effect on the cancer. She is still alive two years after the onset of the disease.

CSF from patient 2, hereafter designed as "CSF2", has the same autoantibodies than CSF1, with a similar staining by immunohistochemitry (table 5):

**Table 5. Immunohistochemistry performed on rat brain section (12 µm thickness, sagittal section) with CSF1, CSF2 and control CSF, showing particular staining pattern**

| | Cerebellum | | | Hippocampus | | | Cortex |
|---|---|---|---|---|---|---|---|
| | Granular layer | Molecular layer | Purkinje cell layer | CA1 cells | CA3 cells | Dentate Gyrus | |
| CSF 1 | + | + | + | + | + | + | + |
| CSF 2 | + | + | + | + | + | + | + |
| Control CSF | - | - | - | - | - | - | - |

A similar staining of two different bands by western-blotting was also observed (table 6):

**Table 6. Western blotting performed with rat brain protein extract showing two specific protein bands recognized by 2 patients' CSF (CSF1 and CSF2) but not by the control CSF**

| | CSF1 | CSF2 | Control CSF |
|---|---|---|---|
| Band at 95 kDa | + | + | - |
| Band at 72 kDa | + | + | - |

Finally CSF 2 recognized both HEK cells expressing TRIM9 and TRIM67 by cell-based assays (CBA) exactly like CSF 1 (table 7)

**Table 7. Antigen confirmation by CBA using HEK cells expressing TRIM9 or TRIM 67**

| | HEK cell expressing TRIM9 | HEK cell expressing TRIM67 |
|---|---|---|
| CSF1 | + | + |
| CSF2 | + | + |
| Control CSFs | - | - |

Immunohistochemistry on double KO mouse brain confirmed that CSF 1 and 2 contained anti-TRIM9/67 antibodies and that they are the unique autoantibodies visible by immunohistochemistry against the central nervous sytem (see results in table 8 below).

**Table 8. Immunohistochemistry on Trim9^{-/-}, Trim67^{-/-} or double KO Trim9&67 ^{-/-} confirming that these two proteins are unique target of patients' autoantibodies**

| | Cerebellum | | | Hippocampus | | |
|---|---|---|---|---|---|---|
| | Granular layer | Molecular layer | Purkinje cell layer | CA1 cells | CA3 cells | Dentate Gyrus |
| WT | +++ | +++ | +++ | +++ | +++ | +++ |
| Trim9 -/- | ++ | ++ | ++ | - | + | - |
| Trim67 -/- | + | + | + | ++ | ++ | ++ |
| Trim 9-/- | - | - | - | - | - | - |
| Trim67 -/- | | | | | | |

Analysis of IgG composition shows that the two CSFs of patients 1 and 2 contain mainly IgG1, even if other IgG subclasses (IgG 2, 3, 4) have also been found, but in a little proportion.

Many DNA constructions coding for different domains of TRIM9 and TRIM67 were used to probe the protein domain containing antibodies' epitope. Results from western-blot experiments show that antibodies in both CSF were polyclonal, since they recognize at least two different epitopes on the TRIM9 protein, one in the RING domain and the other in B-Box, Coiled-coil or FnBD domain but not the B30.2. On the TRIM67 protein, antibodies react strongly with the B-Box domain and the one in C-terminal containing Coiled-coil, FnBD and B30.2 domain. The N-terminal domain of TRIM67 with a lacking RING domain was very weakly recognized by the antibodies.

The technique of "end-point dilution" was employed to estimate antibodies titres in the serum and the CSF of the two positive patients. Serial dilutions of sera or CSFs were incubated with HEK cells expressing either TRIM9 or TRIM67. The presence of antibodies was revealed with a fluorescence-coupled secondary antibody against human IgG. The "end-point dilution" is the dilution ratio at which no fluorescence signal is observed by the experimenter.

Results are presented in table 9 below:

**Table 9. Autoantibodies titers in the serum and CSF of two positive patients**

| | TRIM9-antibodies titer | | TRIM67-antibodies titer | |
|---|---|---|---|---|
| | Serum | CSF | Serum | CSF |
| Patient 1 | 1/128000 | 1/8000 | > 1/256000 | 1/8000 |
| Patient 2 | 1/64000 | 1/4000 | > 1/256000 | 1/8000 |

Both patients TRIM9/67-positive are negative for TRIM46 autoantibodies (results not shown).

In order to look into the pathogenesis mechanism, the effect of patients' CSF on developing murine hippocampal neuron was verified *in vitro.* No significant effect of antibodies on neuron viability and development were observed.

### Example 2. Specificities of TRIM9 and TRIM67 autoantibodies for paraneoplastic cerebellar degeneration

To ensure that anti-TRIM9 and TRIM67 autoantibodies are specific of paraneoplastic cerebellar degeneration, different control groups of patients were tested, using CBA with HEK293 cells expressing TRIM9 or TRIM67. In particular the following samples were tested:
- Sera of 63 patients with small cell lung cancer, without any paraneoplastic neurological syndrome;
- CSF of 100 patients with various autoimmune encephalitis; among them, 29 patients have an associated cancer, and 71 patients do not present any cancer;
- CSF of 165 patients with no inflammatory neurodegenerative diseases (25 with fronto-temporal dementia, 15 Lewy body disease, 20 with psychosis, 15 with communicant hydrocephalus and 90 with Alzheimer's disease).

Results show that the sera and the CSFs issued from patients belonging to these control groups are negative, i.e. they do not contain any antibody specific for TRIM9 and/or TRIM67.

### Example 3. Materials and methods

**Immunohistochemistry:** Freshly prepared adult rat brains were fixed in 4% paraformaldehyde (PFA) for 1h, frozen and sliced into 10 µm-thick sections. Immunolabeling was performed with patients' CSF (1:10) and revealed with peroxidase-conjugated secondary antibody against human IgGs (1:1000, 709-065-149 J, Jackson Immunoresearch, Immunotech, Montluçon, France). For fluorescent labeling, immunostaining was revealed with appropriate Alexa fluorophore-conjugated secondary antibodies (1:1500, A11013, Thermofischer, Courtaboeuf, France).

**Western blot:** Whole adult rat brains were dislocated. The tissue was homogenated in the lysis buffer containing 50 mM Tris-HCl pH 7.5, 150 mM NaCl, 1 mM EDTA, 1% NP-40, 0.5% deoxycholate, 0.1% SDS, orthovanadate, benzonase, phosphatase inhibitor, protease inhibitor. The homogenate was incubated on ice during 30 min and sonicated. Cell debris was discarded by 10 min centrifugation at 10000 g and 4°C. Twenty µg of whole brain protein extract were used for western blotting to screen specific band recognized by patients' CSF (1/100).

**Immunoprecipitation and mass spectrometry-based identification:** A positive CSF (patient 1) and a negative CSF (control CSF) were used. Five µL of CSF were mixed with 50 µL of protein G-conjugated agarose beads (Sigma Aldrich, Lyon, France) and completed to 500 µL with PBS. The mixture was incubated for 2 h at 4°C with rotation to allow for the coupling of CSF's antibodies with protein G. Simultaneously, whole protein extract from one mouse brain was prepared and incubated with 50 µL of agarose beads. Nonspecific contaminant was removed from the lysate by 5-min centrifugation at 16 000 g and 4°C. Cleared lysate was subsequently used for immunoprecipitation with antibodies-conjugated agarose beads.

Immunoprecipitate was analyzed by SDS-PAGE, and this was followed by silver-stain and western blotting. The area between 72 kDa and 95 kDa of a Coomassie-blue stained gel was excised and analyzed by mass spectrometry-based proteomics, as previously described. Briefly, proteins were in-gel digested using modified trypsin (Promega, sequencing grade), and resulting peptides were analyzed by online nanoLC-MS/MS (UltiMate 3000 and LTQ-Orbitrap Velos Pro, Thermo Scientific). Peptides and proteins from different samples were identified, filtered and compared using Mascot and Proline software.

**Cell-based assay (CBA):** To confirm antibodies target, HEK 293 cells were transfected with pCS2-TRIM9 plasmid (Menon et al., 2015) or pCS2-TRIM67 plamid for a transient overexpression. Fixed and permeabilized cells were then immunostained with patients' CSF (1:100) and commercial mouse monoclonal anti-Myc antibody (ref), and then they were revealed with appropriate fluorochrome-conjugated secondary antibodies (1:1500, A11013, and A11001, Thermofischer). Photographs of stained cells were taken with fluorescent microscope Axio Imager Z1 (Carl Zeiss, France). Antibody titers were obtained by using serial dilutions of serum and CSF, as well as full length TRIM9-transfected HEK 293 cells. Patients' antibody IgG subtypes contained in serum or CSF were identified using full length AK5-transfected HEK 293 cells and secondary anti-human antibodies specific for IgG1 (MCA4774, Biorad), IgG2 (555873, BD pharmingen), IgG3 (5247-9850, ABD serotec) and IgG4 (555881, BD pharmingen).

**For epitope mapping on TRIM9,** several plasmids described in (Tanji et al., 2010) coding for different domains were used.

**For epitope mapping on TRIM67,** three constructions coding for N-terminal region (F1), B-Box domain (F2)and C-terminal region containing Coiled-coil, FnBD and B30.2 domain (F3) were generated by PCR (In fusion, Clontech) with the following primers, as shown in table 10:

**Table 10. Primers used for synthesis of protein domains of TRIM67**

| F1 - SEQ ID NO. 1 | F2 - SEQ ID NO. 2 | F3 - SEQ ID NO. 3 |
|---|---|---|
| CGGCGGTGTAGCCT GGTGGCTCCCGCCA GGCTACACCGCCGG GGCTGCAGC | ATTCAATGGCCATCTGCCAGCTGTGCAGATG GCCATTGAATTCAAGTCCTCTTCAGAAAGGT GACCTAGCCTGGTGGCTCCCGCCAGGCTAG GTCACCTTGGTGAGCAGC | ATTCAATGAAAGAGAG GGAGCACAAGCTCTCT TTCATTGAATTCAAGT CCTCTTCAGA |

### REFERENCES

### PATENTS

US 6,387,639

### BIBLIOGRAPHIC REFERENCES

Graus F, Delattre JY, Antoine JC, Dalmau J, Giometto B, Grisold W, Honnorat J, Smitt PS, Vedeler Ch, Verschuuren JJ, Vincent A, Voltz R. Recommended diagnostic criteria for paraneoplastic neurological syndromes. J Neurol Neurosurg Psychiatry. 2004 Aug;75(8):1135-40.
van Coevorden-Hameete MH, van Beuningen SFB, Perrenoud M, Will LM, Hulsenboom E, Demonet JF, Sabater L, Kros JM, Verschuuren JJGM, Titulaer MJ, de Graaff E, Sillevis Smitt PAE, Hoogenraad CC. Antibodies to TRIM46 are associated with paraneoplastic neurological syndromes. Ann Clin Transl Neurol. 2017 Jul 28;4(9):680-686.
Ricchiuti V, Briand JP, Meyer O, Isenberg DA, Pruijn G, Muller S. Epitope mapping with synthetic peptides of 52-kD SSA/Ro protein reveals heterogeneous antibody profiles in human autoimmune sera.Clin Exp Immunol. 1994 Mar;95(3):397-407.
Wolska N, Rybakowska P, Rasmussen A, Brown M, Montgomery C, Klopocki A, Grundahl K, Scofield RH, Radfar L, Stone DU, Anaya JM, Ice JA, Lessard CJ, Lewis DM, Rhodus NL, Gopalakrishnan R, Huang AJ, Hughes PJ, Rohrer MD, Weisman MH, Venuturupalli S, Guthridge JM, James JA, Sivils KL, Bagavant H, Deshmukh US. Brief Report: Patients With Primary Sjögren's Syndrome Who Are Positive for Autoantibodies to Tripartite Motif-Containing Protein 38 Show Greater Disease Severity. Arthritis Rheumatol. 2016 Mar;68(3):724-9. doi: 10.1002/art.39497.
Reymond A, Meroni G, Fantozzi A, Merla G, Cairo S, Luzi L, Riganelli D, Zanaria E, Messali S, Cainarca S, Guffanti A, Minucci S, Pelicci PG, Ballabio A. The tripartite motif family identifies cell compartments. EMBO J. 2001 May 1;20(9):2140-51.
Yaguchi H, Okumura F, Takahashi H, Kano T, Kameda H, Uchigashima M, Tanaka S, Watanabe M, Sasaki H, Hatakeyama S. TRIM67 protein negatively regulates Ras activity through degradation of 80K-H and induces neuritogenesis.J Biol Chem. 2012 Apr 6;287(15):12050-9. doi: 10.1074/jbc.M111.307678. Epub 2012 Feb 15.
LARMAN, H. Benjamin, ZHAO, Zhenming, LASERSON, Uri, et al. Application of a synthetic human proteome to autoantigen discovery through PhIP-Seq. Nature biotechnology, 2011, vol. 29, no 6, p. 535.
HONNORAT, Jérôme et ANTOINE, Jean-Christophe. Paraneoplastic neurological syndromes. Orphanet journal of rare diseases, 2007, vol. 2, no 1, p. 22.
Tanji K, Kamitani T, Mori F, Kakita A, Takahashi H, Wakabayashi K. TRIM9, a novel brain-specific E3 ubiquitin ligase, is repressed in the brain of Parkinson's disease and dementia with Lewy bodies. Neurobiol Dis. 2010 May;38(2):210-8.

## Claims

1. Process *in vitro* for diagnosing a paraneoplastic neurological syndrome (PNS) associated with a tumor in an individual, comprising the detection of at least one antibody chosen among antibodies against TRIM9 and antibodies against TRIM67, in a biological fluid of said individual.

2. Process according to claim 1, wherein the presence of at least one of said antibodies is indicative of the presence of a PNS in said individual.

3. Process *in vitro* for identifying the presence of a tumor in an individual affected by a neurological syndrome, comprising the detection of at least one antibody chosen among antibodies against TRIM9 and antibodies against TRIM67, in a biological fluid of said individual.

4. Process according to claim 3, wherein the presence of at least one of said antibodies is indicative of the presence of a tumor in said individual.

5. Process according to anyone of claims 1 to 4, wherein the tumor is not a melanoma.

6. Process according to anyone of claims 1 to 5, wherein the tumor is a pulmonary tumor, in particular an adenocarcinoma or a small cell lung cancer.

7. Process according to anyone of claims 1 to 6, wherein the neurological syndrome associated with the tumor is at least one of the following syndromes: subacute cerebellar ataxia, opsoclonus-myoclonus, sensory neuronopathy, limbic encephalitis, encephalomyelitis, stiff-person syndrome, dementia, brainstem encephalitis, Lambert-Eaton myasthenic syndrome and dermatomyositis.

8. Process according to anyone of claims 1 to 7, comprising a preliminary step consisting in a step of detection of at least one antibody chosen among the group consisting of: antibodies anti-Yo, anti-Hu, anti-CV2/CRMP5, anti-Ri and anti-Ma2.

9. Process according to claim 8, wherein the antibodies chosen among the group consisting of: antibodies anti-Yo, anti-Hu, anti-CV2/CRMP5, anti-Ri and anti-Ma2 are undetectable in the biological fluid of the individual.

10. Process according to anyone of claims 1 to 9, wherein both antibodies against TRIM9 and TRIM67 are detected in the biological fluid of the individual.

11. Process according to anyone of claims 1 to 10, wherein the biological fluid is chosen among the group consisting of: cerebrospinal fluid, serum, whole blood, urine, lymph, saliva, sputum and tears.

12. Process according to anyone of claims 1 to 11, wherein the detection of antibodies is performed according to at least one of the following techniques: immunoblotting such as western blot or dot-blot, immunohistochemistry, ELISA and cell-based assay.

13. Process according to anyone of claims 1 to 12, wherein the detection of at least one antibody comprises furthermore the quantification of said at least one antibody in the biological fluid.

14. Process for monitoring the evolution of a PNS in an individual affected by this syndrome, comprising :
a. Determining the amount of at least one antibody chosen among antibodies against TRIM9 and antibodies against TRIM67, in a biological fluid of said individual, at a moment T₀;
b. Determining the amount of the same at least one antibody as quantified in step (a), in a biological fluid of said individual, at a moment T₁ later than T₀;
c. Comparing the two amounts obtained at T₀ and T₁,
wherein a decrease, between T₀ and T₁, of the amount of said at least one antibody, is indicative of an improvement in the PNS affecting the individual.

15. Kit of diagnostic comprising:
a. at least one antigen or antigen-expressing cell or nucleic acid chosen among the group consisting of:
i. Cells expressing TRIM9 and/or TRIM67 proteins;
ii. TRIM9 and/or TRIM67 proteins;
iii. TRIM9 and/or TRIM67 antigenic fragments; and
iv. An expression vector carrying at least one nucleic acid encoding TRIM9 and/or TRIM67 proteins, or TRIM9 and/or TRIM67 antigenic fragments;
b. Anti-human IgG antibody coupled to a probe; and
c. Reactants useful for performing the *in vitro* processes according to any one of claims 1 to 14.
